# EUROPEAN PATENT SPECIFICATION

(11) **EP 1 932 485 B1**
(45) Date of publication and mention of the grant of the patent: **19.05.2010**
(21) Application number: 06126187.1
(22) Date of filing: 14.12.2006
(51) Int. Cl.: A61B 17/072, A61B 17/00, A61B 17/064, A61B 17/10, A61B 17/32, A61B 17/34

(54) **Laparoscopic stapling device**
Laparoskopische Klammervorrichtung
Dispositif d'agrafage laparoscopique

(43) Date of publication of application: 18.06.2008
(73) Proprietor: ETHICON ENDO-SURGERY, INC., Cincinnati, OH 45242-2839 (US)
(72) Inventor: Longo, Antonio, 90134, Palermo (IT); Popovic, Drago, Sunrise Beach, Queensland 4567 (AU); Pastorelli, Alessandro, 00136, Roma (IT); D'Arcangelo, Michele, 00142, Roma (IT); Kuhns, Jesse J., Cincinnati, OH 45208 (US); Bilotti, Federico, 04011, Aprilia (Latina) (IT)
(74) Representative: Leihkauf, Steffen Falk

(56) References cited:
- WO-A-2004/110285
- US-A- 4 869 414
- US-A- 5 915 616
- US-A1- 2005 113 821
- US-A1- 2005 143 759
- US-A1- 2006 163 312

## Description

The invention relates to a surgical stapling instrument, which can be used, e.g., in the diagnosis and therapy of all pathologies best treated by a curved stapled resection. Curved staplers have proven to be very effective in cutting and stapling tissue with improved visibility and access over traditional staplers. The contoured shape of the curved end effector makes it possible to better position and visualize the end effector and the tissue intended to be treated. Publications US2005l0143759A1, US2006/163312A1 and WO2004/190285A disclose surgical staplers which form background art for the claimed invention. 1

While known curved staplers are very satisfying in open surgery, they cannot be used laparoscopically due to their general shape and the size of their instrument shaft and staple fastening assemblies which make it impossible or very difficult to introduce the instrument through a laparoscopic access port, such as a trocar port or hand port, to comfortably reach target sites remote from the laparoscopic access port, to handle the instrument during the procedure and to pneumatically seal the laparoscopic access port around the instrument shaft. Moreover, the shapes and dimensions of known staplers are not sufficiently ergonomic for laparoscopic diagnosis and therapy of pathologies.

The present invention addresses at least some of the above and other issues.

According to one aspect of the invention, the surgical stapling instrument comprises a frame having a handle connected to a proximal end of a support shaft and a staple fastening assembly connected to a distal end of the support shaft. Here and in the following, the terms "proximal" and "distal" relate to the user as reference, which means that the parts of the instrument close to the user under normal operating conditions are designated as "proximal" and the parts remote from the user under normal conditions are designated as "distal".

The support shaft comprises a curved shaft segment formed between a proximal shaft segment and a distal shaft segment so that a longitudinal axis of the distal shaft segment and staple fastening assembly is inclined with respect to a longitudinal axis of the proximal shaft segment, thereby improving the handling, visualization and ergonomics of the instrument during laparoscopic operations.

According to an embodiment, the longitudinal axis of the distal shaft segment is inclined to the longitudinal axis of the proximal shaft segment at an angle of about 15° to 75°, preferably of about 35° to 55°, even more preferably at an angle of about 45°.

The staple fastening assembly includes a curved cartridge, which comprises at least one curved open row of staples, and, at the distal end of the instrument, a curved anvil, which is adapted to cooperate with the cartridge for forming the ends of the staples exiting from the cartridge. Particularly, the entire staple fastening assembly is curved about a longitudinal (proximal - distal) axis so that a tissue clamping interface defined by a distal surface of the cartridge and by an opposite proximal staple forming surface of the anvil are substantially arch shaped. Such a curved shape of the staple fastening assembly enables unobstructed access towards the concave inner faces of the cartridge and the anvil and thus to the tissue to be resected. A cartridge moving device is adapted to move the cartridge essentially in parallel relationship towards the anvil from a spaced position for positioning tissue therebetween to a closed position for clamping the tissue. The staples can be driven out of the cartridge towards the anvil by means of a staple driving device.

Herein, the term "staple" is used in a very general sense. It includes metal staples or clips, but also surgical fasteners made of synthetic material and similar fasteners which might have a counterpart (retainer member) held at the anvil.

According to an embodiment, the support shaft is substantially rigid and preferably non-deformable along its entire length from the proximal end which is connected to the handle to the distal end which is connected to the staple fastening assembly.

According to one aspect of the invention, the connection of the proximal end of the support shaft to the handle is rotatably adjustable about the longitudinal axis of the proximal shaft segment. Such a rotatably adjustable connection makes it possible to adjust the orientation of the surgeon's hand holding the handle with respect to the staple fastening assembly.

According to a further aspect of the invention, the support shaft comprises a smooth and continuously convex external surface segment which facilitates pneumatic sealing during laparoscopic use of the instrument. According to a preferred embodiment, the smooth and continuously convex external surface segment has a circular cross-section and extends substantially along the entire length of the support shaft.

In accordance with a further embodiment, the curvatures of the curved staple fastening assembly and the curvature of the support shaft are configured so that the convex sides of the staple fastening assembly and the convex side of the support shaft are on the same side of the instrument or, in other words, the convex sides of the staple fastening assembly and the convex side of the support shaft point approximately in a first direction while the concave sides of both the staple fastening assembly and the support shaft point approximately in a second direction opposite the first direction. This configuration enables the surgeon to position the staple fastening assembly inside the body in a way that a laparoscope inserted into the patient's body through a further laparoscopic access port can easily visualize the concave side of the staple fastening assembly and the tissue positioned or clamped between the cartridge and the anvil.

In accordance with a further aspect of the invention, the proximal shaft segment is substantially straight and has a greater length then the distal shaft segment which may be also at least approximately straight. This enables a deep introduction of the staple fastening assembly and, at the same time, a certain liberty of inclination and lateral movement. Preferably, the length of the proximal shaft segment is greater than the sum of the lengths of the curved shaft segment and the distal shaft segment.

According to an embodiment, a knife is contained within the cartridge and is positioned such that there is at least one row of staples on at least one side of the knife. The knife is moved towards the anvil by means of a knife actuating device. If the stapling instrument does not include a knife, tissue to be resected after stapling can be cut by means of a separate surgical instrument.

According to a further embodiment, the cartridge is detachably connectable to the staple fastening assembly and replaceable so that the instrument can be re-used after firing one cartridge and replacing the spent cartridge by a new cartridge. It is also contemplated that the cartridge is re-usable several times. In this case the cartridge itself is reloadable with staples and, if provided, with a cutting blade or knife.

According to a further aspect of the invention, the outer faces of the cartridge and the anvil are smoothly rounded to provide a gentle touch to the surrounding tissue during laparoscopic introduction and operation of the instrument. The cartridge and the anvil can have a generally arc-like shape in the cross-sectional plane, the arc extending over an angle in the range 90° to 270°.

According to an embodiment, the cartridge moving device includes a movement actuating lever (e.g. at the handle) for actuating the movement of the cartridge and the staple driving device includes a staple driving actuating lever (e.g. at the handle) for "firing" the instrument.

In an embodiment of the invention, the staple fastening assembly is detachably mounted to the distal end of the support shaft. This allows the frame (handle and shaft) of the instrument and many parts of the cartridge moving device, the staple driving device and the knife actuating device to be designed as re-usable components, which are sterilized after each surgical procedure, whereas the staple fastening assembly can be replaced after each surgical procedure.

The main advantage of the surgical stapling instrument according to the invention is its ability to allow laparoscopic tissue resections and stapling under direct laparoscopic visualization, improved laparoscopic access, pneumatic sealing of the laparoscopic access port around the support shaft in case the instrument is inserted through a comparatively wide hand port (permitting the use of a bulky or large staple fastening assembly) or through a trocar port. In the latter case, the external diameter of the staple fastening assembly need to be less than the internal diameter of the trocar port passage channel, e.g. 14mm to 16mm for comparatively small trocars or greater diameters of bigger trocars, and the curvature of the curved shaft segment needs to be such that the support shaft can still be introduced and withdrawn through the trocar port passage channel. Moreover, the surgical stapling instrument provides improved handling, visualization and ergonomics capabilities compared to known devices.

In accordance with a yet further aspect of the invention, the anvil is connected to the staple fastening assembly on one side only and shaped as a hook in order to allow dissection and mobilization of anatomical structures, that is the anvil is laterally cantilevered and curved or bent in a way that it can be used to suspend, hold or pull anatomical structures in order to open a gap between adhering organs (referred to as "dissection") and to remove mesentery from a tissue portion, such as a part of the intestine or a blood vessel before stapling and cutting (referred to as "mobilization"). To this end, a lateral free end of the anvil is preferably shaped as a blunt tip.

According to an embodiment, a distal surface at the free end of the anvil is tapered in the direction of the longitudinal axis of the staple fastening assembly so that the height of the anvil in the direction of the longitudinal axis is reduced towards the free end of the anvil.

In accordance with an embodiment, the tapered distal surface portion is inclined to the proximal staple forming surface of the anvil at an angle of about 30° to 70°, preferably at an angle of about 30° to 60°, yet more preferably at an angle of about 35°.

Such a hook feature of the anvil makes it possible to perform dissection by means of the same instrument that performs stapling and cutting, thereby obviating further traction instruments which in turn increases the visualization space and reduces the number of laparoscopic access ports. A laparoscopic cutter stapler with an anvil configured in the above described manner may also contribute to an easier transection, that is cutting and stapling, of vessels and other anatomical structures.

Even though the above described shape of the anvil is beneficial in combination with a curved support shaft, it is also contemplated to implement such an anvil shape in a laparoscopic cutter stapler with a straight or otherwise shaped support shaft, e.g. to provide a laparoscopic curved cutter stapler which addresses at least some of the drawbacks of known laparoscopic linear endo-cutters which are often difficult to position and which do not allow dissection and require additional instruments to hold the anatomical structures that require stapling.

These and other details and advantages of the present invention shall be made apparent from the accompanying drawings and the description thereof, which illustrate embodiments of the invention and, together with the general description of the invention given above, and the detailed description of the invention given below, serve to explain the principles of the present invention.

Figure 1 is a isometric view of a surgical stapling instrument according to an embodiment of the invention;

Figure 2 is a proximal view of the surgical stapling instrument in figure 1;

Figure 2A is a distal view of a cartridge device of the surgical stapling instrument according to an embodiment of the invention;

Figure 3 is a top view of the surgical stapling instrument in figure 1;

Figure 4 is an isometric view of a staple fastening assembly of the surgical stapling instrument according to an embodiment of the invention;

Figure 5 illustrates a renal vein transection using a known linear endo-cutter stapler;

Figure 6 illustrates a renal vein manipulation using a surgical stapling instrument according to an embodiment of the invention;

Figure 7 illustrates a renal vein dissection and transection using a surgical stapling instrument according to an embodiment of the invention;

Figure 8 illustrates a renal artery dissection and transection using a surgical stapling instrument according to an embodiment of the invention;

Figure 9 illustrates a right ureter transection using a surgical stapling instrument according to an embodiment of the invention;

Figure 10 illustrates a biliary tract mobilization and transection using a surgical stapling instrument according to an embodiment of the invention;

Figure 11 illustrates an appendectomy using a surgical stapling instrument according to an embodiment of the invention;

Figure 12 is a schematic proximal view of a laparoscopic hand port;

Figure 13 is a schematic side view of the laparoscopic hand port in figure 12, fastened to an abdominal incision;

Figure 14 is a schematic longitudinally sectioned view of a laparoscopic trocar port.

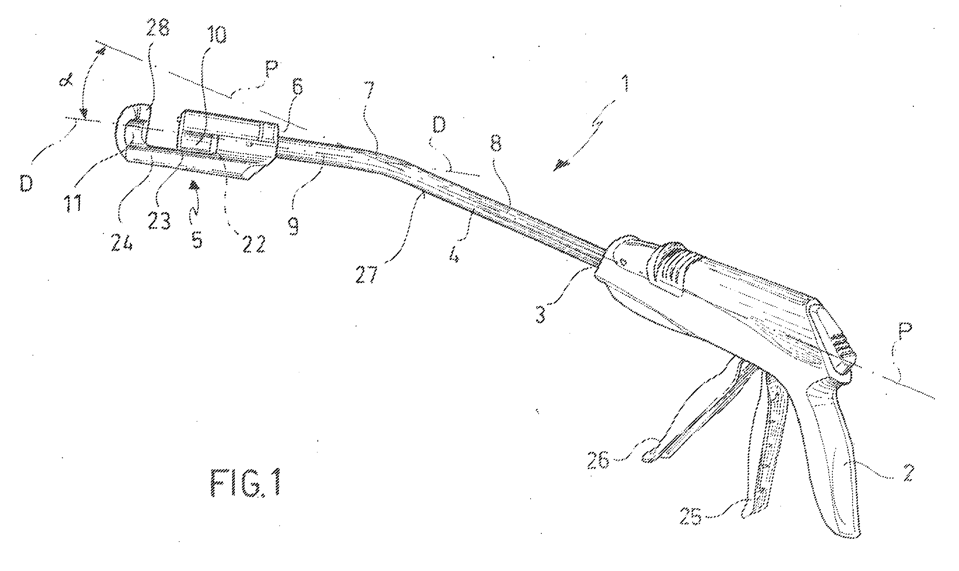

Turning to the figures, fig. 1 is an isometric overall view of a surgical stapling instrument 1 according to an embodiment of the invention.

The surgical stapling instrument 1 comprises a frame having a handle 2 connected to a proximal end 3 of a support shaft 4 and a staple fastening assembly 5 connected to a distal end 6 of the support shaft 4. The support shaft 4 comprises a curved shaft segment 7 formed between a proximal shaft segment 8 and a distal shaft segment 9 so that a longitudinal axis D-D of the distal shaft segment 9 and staple fastening assembly 5 is inclined with respect to a longitudinal axis P-P of the proximal shaft segment 8, thereby improving the handling, visualization and ergonomics of the instrument during laparoscopic operations.

According to the illustrative embodiment, the longitudinal axis D-D of the distal shaft segment 9 is inclined to the longitudinal axis P-P of the proximal shaft segment 8 at an angle α of about 15° to 75°, preferably of about 35° to 55°, even more preferably at an angle of about 45°.

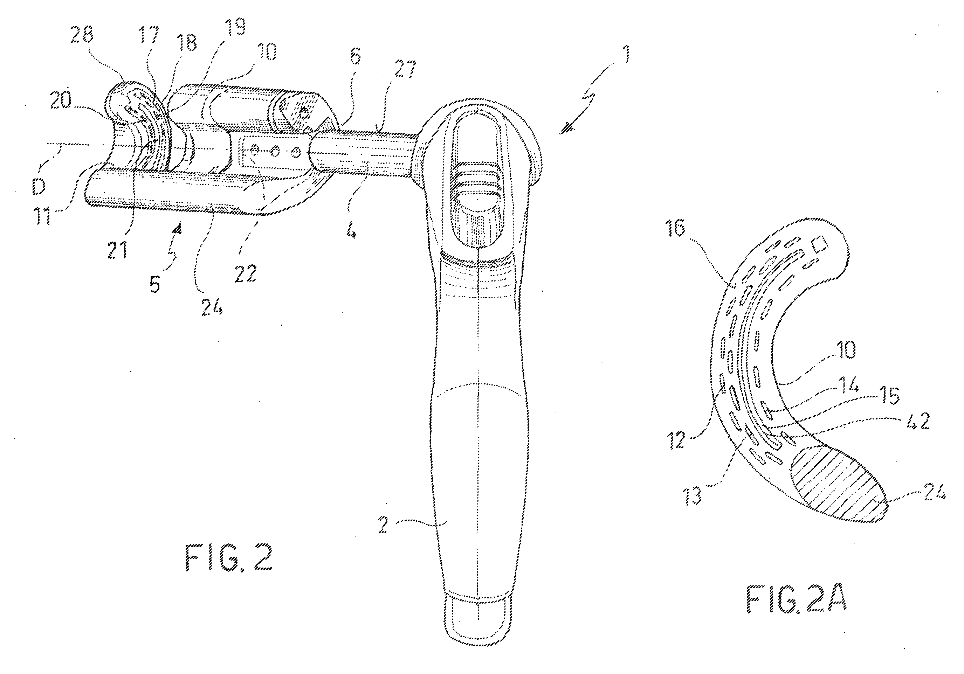

The staple fastening assembly 5 includes a curved cartridge 10 and, at the distal end of the instrument, a curved anvil 11, which is adapted to cooperate with the cartridge 10 for forming the ends of the staples exiting from the cartridge 10. In the embodiment (Fig. 2A) the cartridge 10 defines three curved rows 12, 13, 14 of substantially parallel staple slots, each of which contains a staple (hidden in the figures) and extend parallel to the longitudinal axis D-D of the staple fastening assembly 5 and in the direction of relative movement between the cartridge 10 and the anvil 11. The cartridge 10 further defines a curved knife slot 15 which houses a curved knife 42 and which is arranged so that two rows 12, 13 of staggered and partially overlapping staple slots are disposed on a convex side thereof and a third row of staple slots 14 is arranged at a concave side of the knife 42.

The staple slots and the knife slot extend distally into corresponding openings in a distal end surface 16 of the cartridge 10 wherefrom the knife 42 is advanced and the staples are expelled and pushed distally against a proximal staple forming surface 17 of the anvil 11 when the instrument is "fired". The staple forming surface 17 comprises three curved rows 18, 19, 20 of staple forming depressions facing the corresponding staple slots 12, 13, 14 of the cartridge 10 and adapted to receive and form the distal ends of the staples pressed against them upon "firing". A curved cutting block 21 of preferably plastic material is provided in the staple forming surface 17 between two rows of staple forming recesses and opposite the knife slot 15 of the cartridge 10 so that it interacts with the knife 42 when the latter cuts through the clamped tissue.

A moving device is provided to move the cartridge 10 and the anvil 11 essentially in parallel relationship towards one another from a spaced position for positioning tissue therebetween to a closed position for clamping the tissue.

In accordance with an embodiment, the moving device comprises a cartridge moving device 22 adapted to move the cartridge 10 distally towards a stationary anvil 11. Alternatively, an anvil moving device may be provided which is adapted to approximate the anvil proximally towards a stationary cartridge.

The cartridge moving device 22 comprises a guide 23, which enables a longitudinal movement of the cartridge 10 with respect to a preferably generally C-shaped supporting structure 24 of the staple fastening assembly 5 and a first push member (hidden in the figures) which is interposed between the cartridge 10 and the supporting structure 24 and connected via a first movement transmitter and movement converting or multiplication mechanisms to a manual operating member arranged at or near the handle 2.

In accordance with an embodiment, the first movement transmitter comprises a rotary rod or a tension or compression rod received inside the hollow support shaft 4. A proximal end of the rotary rod is connected via a speed increasing gear (hidden in the figures) to a manual movement actuating lever 25 arranged near the handle 2 and a distal end of the rotary rod cooperates with a threaded portion of a screw drive which meshes a corresponding thread of the push member, so that manual actuation of the lever 25 causes the rotary rod to rotate and the screw drive to move the push member together with the cartridge 10 distally towards the anvil 11 from the open position shown in FIGS. 1 to a closed position. Details of such a mechanism for effecting this movement by means of the lever 25 are described, e.g., in U.S. Pat. No. 5,605,272. During the movement, the guide 23 holds the cartridge 10 in parallel relationship with respect to the staple forming surface 17 of anvil 11.

In order to actually fasten the staples to the clamped tissue and cutting the tissue, a so called staple driving device is provided which comprises a slidably supported second push member (hidden in the figures) with a plurality of staple pushing portions arranged in the staple slots at a proximal side of the staples and a knife pushing portion arranged at the proximal side of the knife 42. The second push member is interposed between the staples and the knife on the one hand and the supporting structure 24 or the cartridge 10 on the other hand, and connected via a second movement transmitter and movement converting or multiplication mechanisms to a second manual operating member arranged at or near the handle 2.

In accordance with an embodiment, also the second movement transmitter comprises a rotary rod or a tension or compression rod received inside the hollow support shaft 4. A proximal end of the rotary rod is connected via a speed increasing gear (hidden in the figures) to a manual staple driving lever 26 arranged near the handle 2 and a distal end of the rotary rod cooperates with a threaded portion of a screw drive which meshes a corresponding thread of the second push member, so that manual actuation of the lever 26 causes the rotary rod to rotate and the screw drive to move the second push member together with the staples and the knife distally towards the anvil 11.

When the staple driving lever 26 is actuated by drawing it towards the handle 2, the staples are expelled from the cartridge 10 and the knife is moved in distal direction as well. The staple driving device is configured in a way that the distal edge of the knife runs behind the pointed ends of the staples so that the knife reaches the tissue clamped between the cartridge 10 and the anvil 11 after the staples have penetrated the tissue. This prevents the tissue from being displaced during the cutting process. After the knife has cut the tissue, the excised tissue specimen contains at least a single row of staples, whereas the tissue remaining in the patient is held approximated by two rows of staggered staples.

A detailed description of an embodiment of a possible staple drive mechanism, is described in U.S. Pat. No. 5,605,272. This document also discloses some safety features which prevent the instrument 1 from being actuated or fired accidentally.

The support shaft 4 is substantially rigid and preferably non-deformable along its entire length from the proximal end 3 which is connected to the handle 2 to the distal end 6 which is connected to the staple fastening assembly 5.

In accordance with an embodiment, the support shaft 4 comprises a substantially tubular profile with a circular cross-section and a smooth and continuously convex external surface segment 27 which facilitates pneumatic sealing during laparoscopic use of the instrument. According to a preferred embodiment, the smooth and continuously convex external surface segment 27 extends substantially along the entire length of the support shaft 4.

In accordance with an embodiment, the curvatures of the curved staple fastening assembly 5 and the curvature of the support shaft 4 are configured so that the convex sides of the staple fastening assembly 5 and the convex side of the support shaft 4 are on the same side of the instrument 1, thereby improving maneuverability of the staple fastening assembly 5 both visualization and access to the clamped tissue.

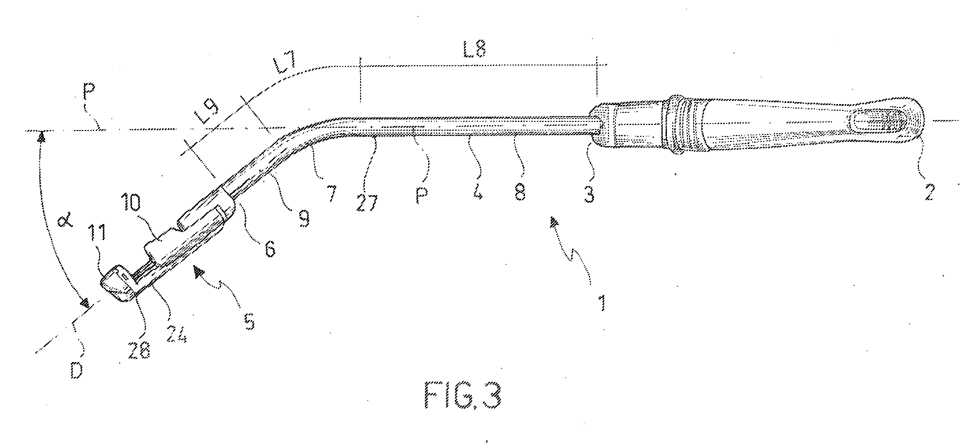

In accordance with the illustrative example in figure 3, the proximal shaft segment 8 is substantially straight and has a length L8 which is greater then the length L9 of the distal shaft segment 9 which may be also at least approximately straight. Moreover, the length L8 of the proximal shaft segment 8 is greater than the sum of the lengths L7 and L9 of the curved shaft segment 7 and the distal shaft segment 9.

According to an embodiment, the connection of the proximal end 9 of the support shaft 4 to the handle 2 is rotatably adjustable about the longitudinal axis P-P of the proximal shaft segment 8. Such a rotatably adjustable connection makes it possible to adjust the orientation of the surgeon's hand holding the handle 2 with respect to the staple fastening assembly 5.

According to a preferred embodiment, the cartridge 10 is detachably connectable to the staple fastening assembly 5 and replaceable so that the instrument 1 can be re-used after firing one cartridge and replacing the spent cartridge by a new cartridge. It is also contemplated that the a single cartridge is re-usable several times. In this case the cartridge itself is reloadable with staples and, if provided, with a cutting blade or knife.

In accordance with a yet further aspect of the invention, the anvil 11 is connected to the staple fastening assembly 5 on one side only and shaped as a hook in order to allow dissection and mobilization of anatomical structures. A free lateral end 28 of the anvil 22 is shaped as a blunt tip and, particularly, a distal surface 29 near the free end 28 of the anvil 11 is tapered in the direction of the longitudinal axis D-D of the staple fastening assembly 5 so that the height of the anvil 11 in the direction of that longitudinal axis D-D is reduced towards the free end 28.

In accordance with an embodiment, the tapered distal surface portion 29 is inclined with respect to the staple forming surface 17 at an angle β of about 30° to 70°, preferably at an angle of about 30° to 60°, yet more preferably at an angle of about 35°.

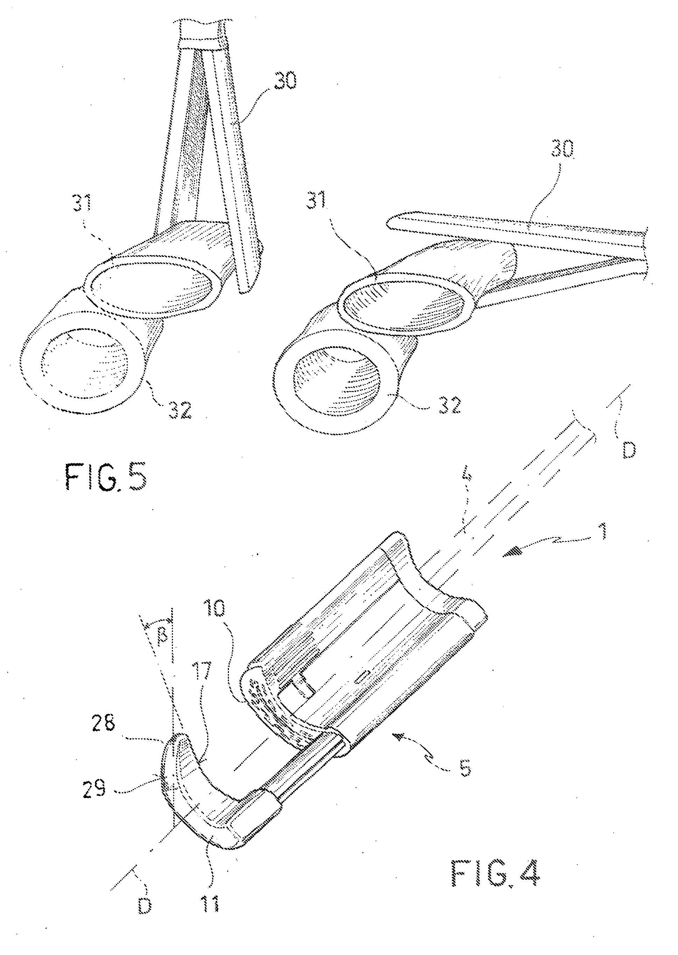

Figure 4 illustrates an embodiment having such a hook shaped anvil which enable the surgeon to perform a laparoscopic dissection by means of the same instrument 1 that performs stapling and cutting. This obviates the need of additional traction instruments for dissection, positioning and mobilization prior to stapling and cutting which are indispensable when performing laparoscopic surgery by means of known endo-cutter staplers 30 (Figure 5). For instance, during a laparoscopic nephrectomy, the known endo-cutter stapler 30 need to be assisted by additional traction and positioning instruments in order to dissect and position the anatomical structures around the target tissue (e.g. a renal vein 31, artery 32 and ureter 33) and to mobilize the target vessel itself.

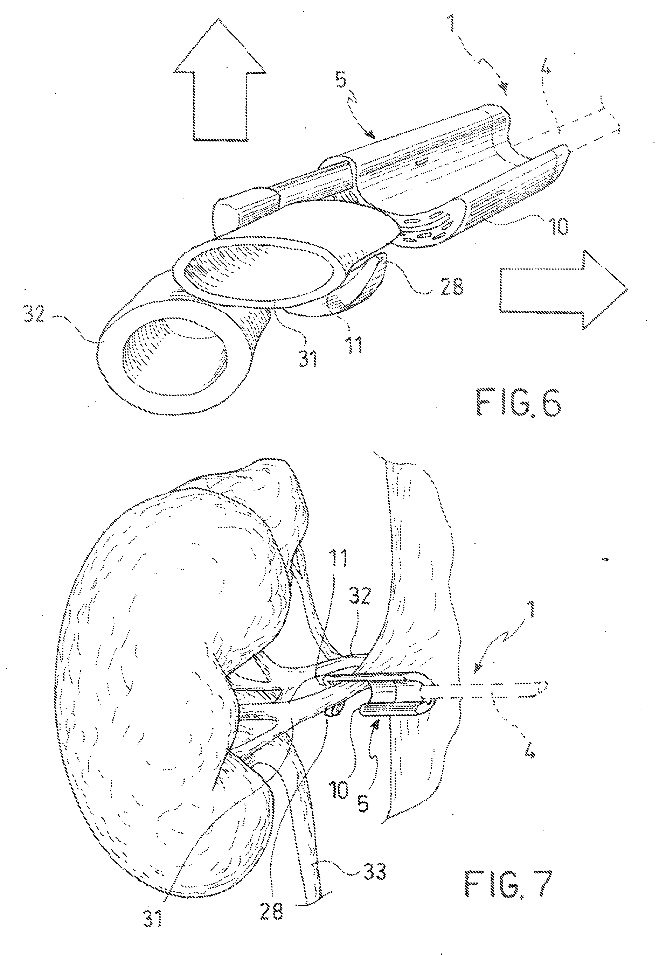

Figure 6 illustrates the instrument 1 while it performs the steps of dissecting the renal vein 31 by hooking the blunt tip of the anvil 11 between the renal vein 31 and artery 32 and gently pushing and tearing the renal vein 31 to allow clear visibility of the adjacent anatomical structure. The arrows indicate possible pushing and tractioning directions of the vein 31 without any need of additional instruments.

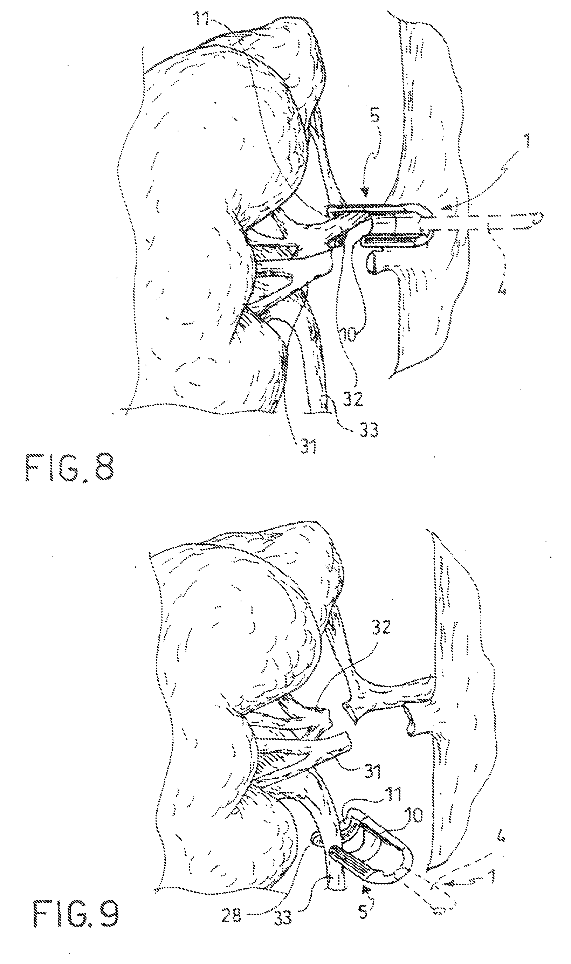

Figure 7 illustrates the instrument 1 during a renal vein dissection and transection, figure 8 illustrates the subsequent step of a renal artery dissection and transection and figure 9 illustrates the final step of right ureter transection by using the same instrument throughout the entire nephrectomy.

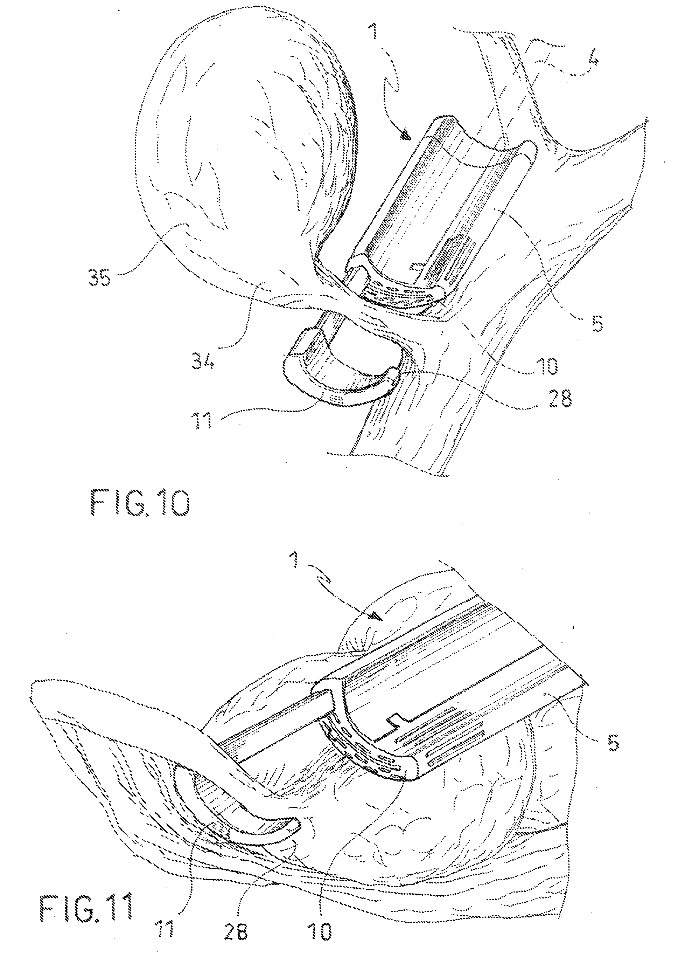

Figure 10 illustrates a further application of the instrument 1 during dissection of the biliary duct 34 of a gall bladder 35 in a laparoscopic biliary intervention prior to stapling and cutting which is performed by the same instrument 1.

Figure 11 illustrates a further application of the instrument 1 during a laparoscopic appendectomy.

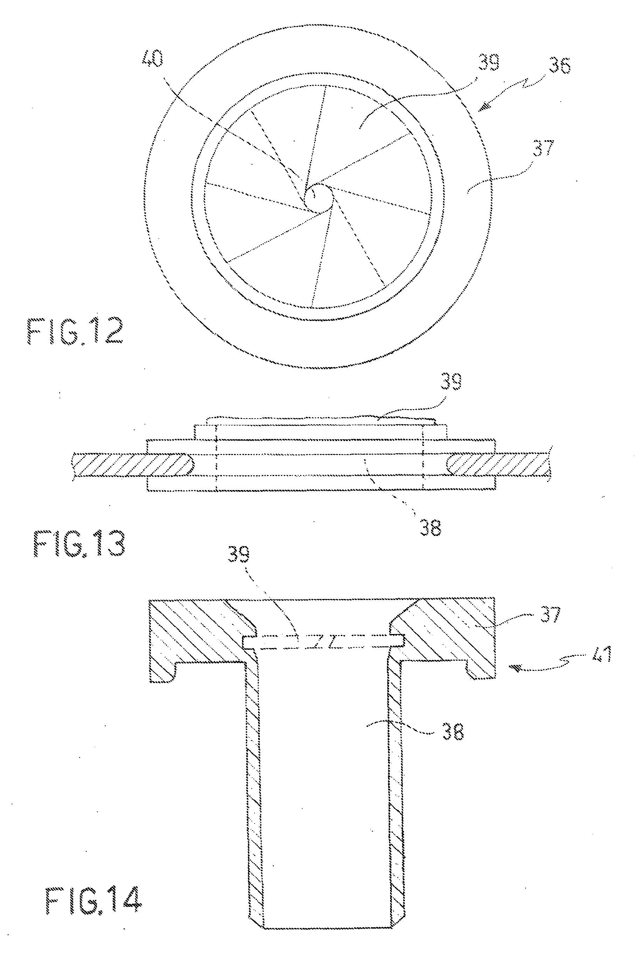

According to a further aspect of the invention, an instrument set for laparoscopic diagnostics and surgery by means of a surgical stapling device is provided which comprises the surgical stapling instrument 1 and a laparoscopic access port device. The laparoscopic access port device, e.g. a laparoscopic hand port 36 (Figures 12 and 13) or a laparoscopic trocar port 41 (Figure 41) includes a ring shaped or tubular frame 37 and a flexible sealing member 39. The frame 37 is adapted to be fastened in an abdominal incision of a patient and defines an internal passage channel 38. The flexible sealing member 39, e.g. a silicone membrane wall having an central opening 40 whose diameter is adjustable by a twisting movement of the membrane wall or, alternatively, a flexible silicone rubber valve, is connected to the passage channel 38. The passage channel 38 and the staple fastening assembly 5 and curved support shaft 4 of the surgical stapling instrument 1 are configured so that the surgical stapling instrument 1 can be inserted through the passage channel 38 into the patient's body. The smooth and convex external surface segment 27 of the support shaft 4 and the flexible sealing member 39 are configured to adhere tightly to one another in order to pneumatically seal the passage channel 38 around the support shaft 4.

The main advantages of the surgical stapling instrument 1 according to the invention is its ability to allow laparoscopic tissue resections and stapling under direct laparoscopic visualization, improved laparoscopic access, pneumatic sealing of the laparoscopic access port around the support shaft in case the instrument is inserted through a comparatively wide hand port (permitting the use of a bulky or large staple fastening assembly) or through a trocar port. In the latter case, the external diameter of the staple fastening assembly need to be less than the internal diameter of the trocar port, e.g. 14mm to 16mm for comparatively small trocars or greater diameters of bigger trocars, and the curvature of the curved shaft segment need to be such that the support shaft can still be introduced end withdrawn through the trocar port. Moreover, the surgical stapling instrument provides improved handling, visualization and ergonomics compared to known devices.

While the present invention has been illustrated by description of several embodiments and while the illustrative embodiments have been described in considerable detail, it is not the intention to restrict or in any way limit the scope of the appended claims to such detail. Additional advantages and modifications may readily appear to those skilled in the art.

## Claims

1. A surgical stapling instrument (1) for laparoscopic manipulation of tissue in a patients body, comprising:
a support shaft (4),
- a handle (2) connected to a proximal end (3) of the support shaft (4),
- a staple fastening assembly (5) connected to a distal end (6) of the support shaft (4) and comprising a curved cartridge (10), which comprises at least one curved open row of staples, and a curved anvil (11), which is adapted to cooperate with the cartridge (10) for clamping the tissue and forming the ends of the staples exiting from the cartridge (10),
wherein the support shaft (4) is substantially rigid and non-deformable along its entire length, **characterized in that** the support shaft (4) comprises a curved shaft segment (7) formed between a proximal shaft segment (8) and a distal shaft segment (9) so that a longitudinal axis (D-D) of the distal shaft segment (9) and staple fastening assembly (5) is inclined with respect to a longitudinal axis (P-P) of the proximal shaft segment (8), and **in that** the curvatures of the curved staple fastening assembly (5) and the curvature of the support shaft (4) are configured so that the convex sides of the staple fastening assembly (5) and the convex side of the support shaft (4) are substantially on the same side of the instrument (1).

2. A surgical stapling instrument (1) according to claim 1, wherein the longitudinal axis (D-D) of the distal shaft segment (9) is inclined to the longitudinal axis (P-P) of the proximal shaft segment (8) at an angle (α) of about 15° to 75°, preferably of about 35° to 55°, even more preferably at an angle of about 45°.

3. A surgical stapling instrument (1) according to claim 1 or 2, wherein the support shaft (4) is substantially rigid and non-deformable along its entire length from the proximal end (3) to the distal end (6).

4. A surgical stapling instrument (1) according to any of the preceding claims, wherein the connection of the proximal end (3) of the support shaft (4) to the handle (2) is rotatably adjustable about the longitudinal axis (P-P) of the proximal shaft segment (8).

5. A surgical stapling instrument (1) according to any of the preceding claims, wherein the support shaft (4) comprises a smooth and continuously convex external surface segment (27) adapted to facilitate pneumatic sealing during laparoscopic use of the instrument (1).

6. A surgical stapling instrument (1) according to the preceding claim, wherein the smooth and continuously convex external surface segment (27) has a circular cross-section and extends substantially along the entire length of the support shaft (4).

7. A surgical stapling instrument (1) according to any of the preceding claims, wherein the curvatures of the curved staple fastening assembly (5) and the curvature of the support shaft (4) are configured so that the convex sides of the staple fastening assembly (5) and the convex side of the support shaft (4) are substantially on the same side of the instrument (1).

8. A surgical stapling instrument (1) according to any of the preceding claims, wherein the proximal shaft segment (8) is substantially straight and has a greater length (L8) then the length (L9) of the distal shaft segment (9).

9. A surgical stapling instrument (1) according to the preceding claim, wherein the length (L8) of the proximal shaft segment (8) is greater than the sum of the lengths (L7, L9) of the curved shaft segment (7) and the distal shaft segment (9).

10. A surgical stapling instrument (1) according to any of the preceding claims, wherein the anvil (11) is connected to the staple fastening assembly (5) on one side only and shaped as a laterally cantilevered hook.

11. A surgical stapling instrument (1) according to the preceding claim, wherein a free end (28) of the anvil (11) is shaped as a blunt tip.

12. A surgical stapling instrument (1) according to any of the preceding claims, wherein a distal surface portion (29) near the free end (28) of the anvil (11) is tapered in the direction of the longitudinal axis of the staple fastening assembly (5) so that the height of the anvil (11) in the direction of said longitudinal axis is reduced towards said free end (28).

13. A surgical stapling instrument (1) according to the preceding claim, wherein the tapered distal surface portion (29) is inclined to a proximal staple forming surface (17) of the anvil (11) at an angle of about 30° to 70°, preferably at an angle of about 30° to 60°, yet more preferably at an angle of about 35°.

14. An instrument set (1, 36, 41) for laparoscopic diagnostics and surgery by means of a surgical stapling device comprising:
- a surgical stapling instrument (1) according to any of the preceding claims and
- a laparoscopic access port device (37; 41) including:
- a ring shaped or tubular frame (37) defining an internal passage channel (38) and adapted to be fastened in an abdominal incision of a patient,
- a flexible sealing member (39),
wherein the passage channel (38) of the laparoscopic access port (37; 41) and the staple fastening assembly (5) and the curved support shaft (4) of the surgical stapling instrument (1) are configured so that the surgical stapling instrument (1) can be inserted through the passage channel (38),
wherein a smooth and convex external surface segment (27) of the support shaft (4) and said flexible sealing member (39) are configured to adhere tightly to one another in order to seal said passage channel (38); around said support shaft (4).

15. An instrument set according to the preceding claim; wherein said laparoscopic access port comprises a laparoscopic trocar port (41) or a laparoscopic hand port (36).

## Patentansprüche

1. Chirurgisches Klammerinstrument (1) zur laparoskopischen Manipulation von Gewebe im Körper eines Patienten, umfassend:
- einen Halteschaft (4),
- einen Griff (2), der mit einem proximalen Ende (3) des Halteschafts (4) verbunden ist,
- eine Klammerbefestigungsanordnung (5), die mit einem distalen Ende (6) des Halteschafts (4) verbunden ist und eine gekrümmte Kassette (10), die wenigstens eine gekrümmte offene Reihe von Klammern umfasst, und einen gekrümmten Amboss (11) umfasst, der dazu angepasst ist, mit der Kassette (10) zum Klemmen des Gewebes und zum Formen der Enden der Klammern, die aus der Kassette (10) austreten, zusammenzuwirken,
wobei der Halteschaft (4) im Wesentlichen starr und nicht-verformbar entlang seiner gesamten Länge ist,
**dadurch gekennzeichnet, dass** der Halteschaft (4) ein gekrümmtes Schaftsegment (7) umfasst, das zwischen einem proximalen Schaftsegment (8) und einem distalen Schaftsegment (9) so gebildet ist, dass eine longitudinale Achse (D-D) von dem distalen Schaftsegment (9) und der Klammerbefestigungsanordnung (5) mit Bezug zu einer longitudinalen Achse (P-P) von dem proximalen Schaftsegment (8) geneigt ist, und dass die Krümmungen der gekrümmten Klammerbefestigungsanordnung (5) und die Krümmung des Halteschafts (4) so konfiguriert sind, dass die konvexen Seiten der Klammerbefestigungsanordnung (5) und die konvexe Seite des Halteschafts (4) im Wesentlichen auf derselben Seite des Instruments (1) sind.

2. Chirurgisches Klammerinstrument (1) nach Anspruch 1, wobei die longitudinale Achse (D-D) von dem distalen Schaftsegment (9) zu der longitudinalen Achse (P-P) von dem proximalen Schaftsegment (8) mit einem Winkel (alpha) von etwa 15° bis 75° geneigt ist, vorzugsweise von etwa 35° bis 55°, noch bevorzugter mit einem Winkel von etwa 45°.

3. Chirurgisches Klammerinstrument (1) nach Anspruch 1 oder 2, wobei der Halteschaft (4) im Wesentlichen starr und nicht-verformbar entlang seiner gesamten Länge von dem proximalen Ende (3) zu dem distalen Ende (6) ist.

4. Chirurgisches Klammerinstrument (1) nach einem der vorhergehenden Ansprüche, wobei die Verbindung von dem proximalen Ende (3) von dem Halteschaft (4) zu dem Griff (2) um die longitudinale Achse (P-P) von dem proximalen Schaftsegment (8) drehbar anpassbar ist.

5. Chirurgisches Klammerinstrument (1) nach einem der vorhergehenden Ansprüche, wobei der Halteschaft (4) ein glattes und kontinuierlich konvexes Außenflächenelement (27) umfasst, das dazu angepasst ist, ein pneumatisches Dichten während einer laparoskopischen Benutzung des Instruments (1) zu erleichtern.

6. Chirurgisches Klammerinstrument (1) nach dem vorhergehenden Anspruch, wobei das glatte und kontinuierlich konvexe Außenflächenelement (27) einen kreisförmigen Querschnitt aufweist und sich im Wesentlichen entlang der gesamten Länge des Halteschafts (4) erstreckt.

7. Chirurgisches Klammerinstrument (1) nach einem der vorhergehenden Ansprüche, wobei die Krümmungen der gekrümmten Klammerbefestigungsanordnung (5) und die Krümmung des Halteschafts (4) so konfiguriert sind, dass die konvexen Seiten der Klammerbefestigungsanordnung (5) und die konvexe Seite des Halteschafts (4) im Wesentlichen auf derselben Seite des Instruments (1) sind.

8. Chirurgisches Klammerinstrument (1) nach einem der vorhergehenden Ansprüche, wobei das proximale Schaftsegment (8) im Wesentlichen gerade ist und eine Länge (L8) aufweist, die größer ist als die Länge (L9) von dem distalen Schaftsegment (9).

9. Chirurgisches Klammerinstrument (1) nach dem vorhergehenden Anspruch, wobei die Länge (L8) des proximalen Schaftsegments (8) größer ist als die Summe der Längen (L7, L9) von dem gekrümmten Schaftsegment (7) und dem distalen Schaftsegment (9).

10. Chirurgisches Klammerinstrument (1) nach einem der vorhergehenden Ansprüche, wobei der Amboss (11) nur an einer Seite mit der Klammerbefestigungsanordnung (5) verbunden ist und als ein seitlich freitragender Haken geformt ist.

11. Chirurgisches Klammerinstrument (1) nach dem vorhergehenden Anspruch, wobei ein freies Ende (28) des Ambosses (11) als stumpfe Spitze geformt ist.

12. Chirurgisches Klammerinstrument (1) nach einem der vorhergehenden Ansprüche, wobei ein distaler Flächenabschnitt (29) nahe dem freien Ende (28) des Ambosses (11) in die Richtung von der longitudinalen Achse der Klammerbefestigungsanordnung (5) verjüngt ist, so dass die Höhe des Ambosses (11) in die Richtung von der longitudinalen Achse zu dem freien Ende (28) hin reduziert ist.

13. Chirurgisches Klammerinstrument (1) nach dem vorhergehenden Anspruch, wobei der verjüngte distale Flächenabschnitt (29) zu einer proximalen Klammerfarmfläche (17) des Ambosses (11) mit einem Winkel von etwa 30° bis 70° geneigt ist, vorzugsweise mit einem Winkel von etwa 30° bis 60°, noch bevorzugter mit einem Winkel von etwa 35°.

14. Instrumentensatz (1, 36, 41) zur laparoskopischen Diagnose und Chirurgie mittels einer chirurgischen Klammervorrichtung, umfassend:
- ein chirurgisches Klammerinstrument (1) nach einem der vorhergehenden Ansprüche und
- eine laparoskopische Zugangsanschfussvorrichtung (37; 41), umfassend:
- einen ringförmigen oder röhrenartigen Rahmen (37), der einen Innenpassagekanal (38) definiert und dazu angepasst ist, in einem Bauchschnitt eines Patienten befestigt zu werden,
- ein flexibles Dichtungselement (39),
wobei der Passagekanal (38) des laparoskopischen Zugangsanschlusses (37; 41) und die Klammerbefestigungsanordnung (5) und der gekrümmte Halteschaft (4) des chirurgischen Klammerinstruments (1) so konfiguriert sind, dass das chirurgische Klammerinstrument (1) durch den Passagekanal (38) eingeführt werden kann,
wobei ein glattes und konvexes Außenflächensegment (27) des Halteschafts (4) und das flexible Dichtungselement (39) dazu konfiguriert sind, fest aneinander anzuhaften, um den Passagekanal (38) um den Halteschaft (4) herum abzudichten.

15. Instrumentensatz nach dem vorhergehenden Anspruch, wobei der laparoskopische Zugangsanschluss einen laparoskopischen Trokar-Anschluss (41) oder einen laparaskopischen Hand-Anschluss (36) umfasst.

## Revendications

1. Instrument d'agrafage chirurgical (1) pour la manipulation laparoscopique du tissu dans le corps d'un patient, comprenant :
une tige de support (4),
une poignée (2) raccordée à une extrémité proximale (3) de la ligne de support (4),
un ensemble de fixation d'agrafe (5) raccordé à une extrémité distale (6) de la tige de support (4) et comprenant une cartouche incurvée (10) qui comprend au moins une rangée d'agrafes ouverte incurvée et une enclume incurvée (11) qui est adaptée pour coopérer avec la cartouche (10) pour bloquer le tissu et former les extrémités des agrafes sortant de la cartouche (10),
dans lequel la tige de support (4) est sensiblement rigide et non déformable le long de toute sa longueur, **caractérisé en ce que** la tige de support (4) comprend un segment de tige incurvé (7) formé entre un segment de tige proximal (8) et un segment de tige distal (9) de sorte qu'un axe longitudinal (D-D) du segment de tige distal (9) et l'ensemble de fixation d'agrafe (5) est incliné par rapport à un axe longitudinal (P-P) du segment de tige proximal (8) et **en ce que** les courbures de l'ensemble de fixation d'agrafe incurvé (5) et la courbure de la tige de support (4) sont configurées de sorte que les côtés convexes de l'ensemble de fixation d'agrafe (5) et le côté convexe de la tige de support (4) sont sensiblement du même côté de l'instrument (1).

2. Instrument d'agrafage chirurgical (1) selon la revendication 1, dans lequel l'axe longitudinal (D-D) du segment de tige distal (9) est incliné par rapport à l'axe longitudinal (P-P) du segment de tige proximal (8) selon un angle (α) d'environ 15° à 75°, de préférence d'environ 35° à 55°, encore de préférence selon un angle d'environ 45°.

3. Instrument d'agrafage chirurgical (1) selon la revendication 1 ou 2, dans lequel la tige de support (4) est sensiblement rigide et non déformable le long de toute sa longueur de l'extrémité proximale (3) à l'extrémité distale (6).

4. Instrument d'agrafage chirurgical (1) selon l'une quelconque des revendications précédentes, dans lequel le raccordement de l'extrémité proximale (3) de la tige de support (4) à la poignée (2) est ajustable de manière rotative autour de l'axe longitudinal (P-P) du segment de tige proximal (8).

5. Instrument d'agrafage chirurgical (1) selon l'une quelconque des revendications précédentes, dans lequel la tige de support (4) comprend un segment de surface externe lisse et convexe de manière continue (27) adapté pour faciliter l'étanchéité pneumatique pendant l'utilisation laparoscopique de l'instrument (1).

6. Instrument d'agrafage chirurgical (1) selon les revendications précédentes, dans lequel le segment de surface externe lisse et convexe de manière continue (27) a une section transversale circulaire et s'étend sensiblement le long de toute la longueur de la tige de support (4).

7. Instrument d'agrafage chirurgical (1) selon l'une quelconque des revendications précédentes, dans lequel les courbures de l'ensemble de fixation d'agrafe incurvé (5) et la courbure de la tige de support (4) sont configurées de sorte que les côtés convexes de l'ensemble de fixation d'agrafe (5) et le côté convexe de la tige de support (4) sont sensiblement du même côté de l'instrument (1).

8. Instrument d'agrafage chirurgical (1) selon l'une quelconque des revendications précédentes, dans lequel le segment de tige proximal (8) est sensiblement droit et a une plus grande longueur (L8) que la longueur (L9) du segment de tige distal (9).

9. Instrument d'agrafage chirurgical (1) selon la revendication précédente, dans lequel la longueur (L8) du segment de tige proximal (8) est plus grande que la somme des longueurs (L7, L9) du segment de tige incurvé (7) et du segment de tige distal (9).

10. Instrument d'agrafage chirurgical (1) selon l'une quelconque des revendications précédentes, dans lequel l'enclume (11) est raccordée à l'ensemble de fixation d'agrafe (5) uniquement sur un côté et formée comme un crochet latéralement en porte à faux.

11. Instrument d'agrafage chirurgical (1) selon les revendications précédentes, dans lequel une extrémité libre (28) de l'enclume (11) est formée comme une pointe émoussée.

12. Instrument d'agrafage chirurgical (1) selon l'une quelconque des revendications précédentes, dans lequel une partie de surface distale (29) à proximité de l'extrémité libre (28) de l'enclume (11) est progressivement rétrécie dans la direction de l'axe longitudinal de l'ensemble de fixation d'agrafe (5) de sorte que la hauteur de l'enclume (11) dans la direction dudit axe longitudinal est réduite vers ladite extrémité libre (28).

13. Instrument d'agrafage chirurgical (1) selon les revendications précédentes, dans lequel la partie de surface distale progressivement rétrécie (29) est inclinée vers une surface de formation d'agrafe proximale (17) de l'enclume (11) selon un angle d'environ 30° à 70°, de préférence selon un angle d'environ 30° à 60°, encore de préférence selon un angle d'environ 35°.

14. Ensemble d'instrument (1,36,41) pour le diagnostic laparoscopique et la chirurgie au moyen d'un dispositif d'agrafage chirurgical comprenant :
un instrument d'agrafage chirurgical (1) selon l'une quelconque des revendications précédentes, et
un dispositif d'orifice d'accès laparoscopique (37 ; 41) comprenant :
un châssis tubulaire ou de forme annulaire (37) définissant un canal de passage interne (38) et adapté pour être fixé dans une incision abdominale d'un patient,
un élément d'étanchéité souple (39),
dans lequel le canal de passage (38) de l'orifice d'accès laparoscopique (37 ; 41) et l'ensemble de fixation d'agrafe (5) et la tige de support incurvée (4) de l'instrument d'agrafage chirurgical (1) sont configurés de sorte que l'instrument d'agrafage chirurgical (1) peut être inséré à travers le canal de passage (38),
dans lequel un segment de surface externe lisse et convexe (27) de la tige de support (4) et ledit élément d'étanchéité souple (39) sont configurés pour adhérer de manière hermétique l'un sur l'autre pour réaliser l'étanchéité dudit canal de passage (38) autour de ladite tige de support (4).

15. Ensemble d'instrument selon la revendication précédente, dans lequel ledit orifice d'accès laparoscopique comprend un orifice de trocart laparoscopique (41) ou un orifice laparoscopique manuel (36).
